# EUROPEAN PATENT APPLICATION

(11) **EP 4 806 348 A1**
(43) Date of publication of application: **16.09.2026**
(21) Application number: 25163478.8
(22) Date of filing: 13.03.2025
(51) Int. Cl.: A61B 5/145, A61B 5/1486, A61B 5/00, A61B 17/34

(54) **SENSOR IMPLANTATION MECHANISM**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application relates to the technical field of medical equipment, and disclosed thereby is a sensor implantation mechanism, which is configured to achieve the repeated use of auxiliary implantation devices. The sensor implantation mechanism comprises a sensor module and a reusable auxiliary implantation device. The sensor module comprises a packaging box, a module support, a guide needle, and a sensor device. The packaging box comprises a bottom shell and a box cover, and the box cover is detachably closed to the top of the bottom shell. The reusable auxiliary implantation device comprises an injection structure and a reset structure, and the injection structure can be fixed to the module support. The injection structure can drive the sensor device to be implanted into a human body. The injection structure is provided with a clamping component, which is configured to clamp the guide needle. When the injection structure moves in an opposite direction of the injection direction, the injection structure is also configured to drive the guide needle to detach from the sensor device. The reset structure is configured to drive a second engagement structure to detach from a first engagement structure and also to drive the guide needle to detach from the clamping component.

## Description

### TECHNICAL FIELD

The present application relates to the technical field of medical equipment, and particularly to a sensor implantation mechanism.

### Background

With the gradual improvement of residents' living standards, people are increasingly concerned about their own health status. Based on this, human body sensor devices are also increasingly being applied in people's daily lives. The human body sensor device is implanted into the user's surface skin during use, and the blood in the user's body is detected by using the sensor device, thereby calculating the user's health indicators.

At present, when implanting human body sensor devices into the human body, auxiliary implantation devices are usually required. However, the existing auxiliary implantation devices are generally disposable, i.e., after being used once, they cannot be reused and can only be discarded, which increases the overall cost of the product and easily pollutes the environment.

### SUMMARY

The present application provides a sensor implantation mechanism that enables the repeated use of implanted devices, thereby reducing product costs and protecting the environment.

The present application provides a sensor implantation mechanism, comprising a sensor module and a reusable auxiliary implantation device;
the sensor module comprises a packaging box, a module support, a guide needle, and a sensor device, the packaging box comprises a bottom shell and a box cover, and the box cover is detachably closed to the top of the bottom shell;
the module support, the guide needle, and the sensor device are all arranged inside the bottom shell, and the module support is detachably connected to the bottom shell; the sensor device is detachably connected to the module support, and the guide needle is detachably installed on the sensor device;
the reusable auxiliary implantation device comprises an injection structure and a reset structure, the injection structure is provided with a first engagement structure, and the module support is provided with a second engagement structure; the first engagement structure is configured to engage with the second engagement structure to fix the module support to the injection device, and the first engagement structure is configured to drive the module support to detach from the bottom shell;
the injection structure can move along an injection direction to drive the sensor device to be implanted into a human body; the injection structure can also move in an opposite direction of the injection direction to drive the module support to detach from the sensor device and make the sensor device stay in the human body;
the injection structure is provided with a clamping component, which is configured to clamp the guide needle; when the injection structure moves in the opposite direction of the injection direction, the injection structure is also configured to drive the guide needle to detach from the sensor device;
the reset structure is configured to drive the second engagement structure to detach from the first engagement structure, so that the module support can detach from the injection structure, and the reset structure is also configured to drive the guide needle to detach from the clamping component.

The sensor implantation mechanism in the present application is to design the reusable auxiliary implantation device and sensor module as two independent structures. When it is necessary to implant the sensor device into the user's body, the box cover of the packaging box can be opened, and the module support and injection structure inside the packaging box can be fixed through the first and second engagement structures, and the guide needle can be clamped on the clamping component. During this process, the packaging box and module support are separated, so that the module support, sensor device, and guide needle can be transferred to the injection structure. Subsequently, the sensor device is implanted into the human body using the injection structure, and the module support is separated from the sensor device. The guide needle is then extracted from the sensor device. Finally, the reset structure is configured to drive the second engagement structure to detach from the first engagement structure, separating the module support from the injection structure, and driving the guide needle to detach from the clamping component, restoring the injection structure to its unused state. When it is necessary to implant the sensor device into the human body next time, a new sensor module can be taken, and the module support can be connected to the injection structure to complete the implantation of the sensor device. Thus, the auxiliary implantation device of the sensor implantation mechanism in the present application can be reused, which not only reduces product costs but also benefits environmental protection.

In some optional embodiments, the reusable auxiliary implantation device further comprises an outer shell, a lower cover, and a main support; the outer shell is sleeved and connected to the main support, and the lower cover is detachably closed to the bottom of the outer shell;
the injection structure comprises a needle return support, an inner sleeve sleeved and connected to the needle return support, and a button module;
the engagement structure is provided at an inner wall of the inner sleeve;
the main support is sleeved on the inner sleeve, and a first limiting structure is provided between the inner sleeve and the main support; when the first limiting structure is in a limiting state, the inner sleeve is relatively fixed to the main support; when the first limiting structure is in an unlocked state, the inner sleeve can move relative to the main support along an axis direction of the inner sleeve, wherein the axis direction of the inner sleeve is consistent with the injection direction;
the button module is provided on the outer shell, and the button module comprises a button body; the button body can move relative to the outer shell along the axis direction of the inner sleeve to drive the first limiting structure to switch from the limiting state to the unlocked state, so as to move the inner sleeve relative to the main support and drive the sensor device to be implanted into the human body.

In some optional embodiments, the first engagement structure comprises a top rib arranged on the inner wall of the inner sleeve, and the top rib is arranged near the bottom of the inner sleeve;
the second engagement structure comprises a first support buckle arranged on the module support, and when the first engagement structure is engaged with the second engagement structure, the first support buckle is pressed against the top rib.

In some optional embodiments, the reset structure comprises a lower cover buckle located inside the lower cover, and the module support is provided with a snap-fit structure;
when the first engagement structure is engaged with the second engagement structure, the lower cover can be assembled with the outer shell from the bottom of the outer shell, so that the lower cover buckle is held against the snap-fit structure;
when the lower cover is detached from the outer shell in a state where the lower cover buckle is held against the snap-fit structure, the lower cover buckle is configured to drive the first support buckle to detach from the top rib, so as to detach the module support from the inner sleeve.

In some optional embodiments, the inner wall of the bottom shell is provided with a clamping structure, which is configured to engage with the second engagement structure to fix the module support relative to the bottom shell;
when the first engagement structure is engaged with the second engagement structure, the first engagement structure is configured to drive the clamping structure to detach from the clamping structure, so as to detach the module support from the bottom shell.

In some optional embodiments, the clamping component comprises two clamping rods, which are symmetrically arranged about the axis of the needle return support, and the first end of the clamping rod is connected to the inner wall of the needle return support;
the clamping rod is inclined to the axis of the needle return support, so that the second ends of the two clamping rods are close to each other, and the second ends of the two clamping rods cooperate to clamp the guide needle.

In some optional embodiments, the reset structure comprises a needle return mandril located inside the lower cover; when the first engagement structure is engaged with the second engagement structure, the lower cover can be assembled with the outer shell from the bottom of the outer shell; the needle return mandril is configured to drive the second ends of the two clamping rods to move away from each other, so as to detach the guide needle from the clamping component.

In some optional embodiments, the reset structure comprises a button mandril connected to the button body, and the button mandril extends along the axis direction of the inner sleeve;
when the button body moves along the axis direction of the inner sleeve, the button mandril can extend between the two clamping rods and drive the second ends of the two clamping rods to move away from each other, so as to detach the guide needle from the clamping component.

In some optional embodiments, a first gear and a second gear are provided between the button module and the outer shell;
when the button module and the outer shell are in the first gear, the button module is configured to drive the first limiting structure to switch from the limiting state to the unlocked state;
when the button module and the outer shell are in the second gear, the button module is configured to drive the second ends of the two clamping rods away from each other.

In some optional embodiments, the button module further comprises a button limiting rib, and the inner wall of the outer shell is provided with a button limiting table;
the outer shell can rotate around the axis of the main support relative to the button module; when the button module and the outer shell are in the first gear, the button limiting table is located below the button limiting rib; when the button module and the outer shell are in the second gear, the button limiting table is offset from the button limiting rib along the circumference of the outer shell.

In some optional embodiments, a main spring is connected between the main support and the inner sleeve, and when the first limiting structure is in a limiting state, the main spring is in a compressed state.

In some optional embodiments, a second limiting structure is provided between the needle return support and the inner sleeve; when the second limiting structure is in a limiting state, the needle return support and the inner sleeve are relatively fixed; when the second limiting structure is in an unlocked state, the needle return support can move relative to the inner sleeve along the axis direction of the inner sleeve.

In some optional embodiments, the inner wall of the main support is provided with a third limiting structure, which is located near the bottom of the main support;
the third limiting structure is configured to limit the movement distance of the inner sleeve relative to the main support, and is also configured to drive the second limiting structure to switch from the locked state to the unlocked state.

In some optional embodiments, the second limiting structure comprises a needle return buckle provided on the outer wall of the needle return support and a second sleeve buckle provided on the inner sleeve, and when the second limiting structure is in a limiting state, the needle return buckle is pressed against the bottom of the second sleeve buckle;
the third limiting structure comprises a support hook provided on the inner wall of the main support; when the inner sleeve moves away from the button module relative to the main support, the support hook is configured to drive the needle return buckle to release from the second sleeve buckle and make the support hook press against the bottom of the first sleeve buckle.

In some optional embodiments, a needle return spring is connected between the inner sleeve and the needle return support; when the second limiting structure is in a limiting state, the needle return spring is in a compressed state.

In some optional embodiments, the inner sleeve is provided with a sleeve limiting rib, and the module support is provided with a limiting groove; when the first engagement structure is engaged with the second engagement structure, the sleeve limiting rib is located in the limiting groove.

In some optional embodiments, the module support is provided with positive and negative buckles, and the sensor device is provided with positive and negative slots;
the positive buckle is clamped to the positive slot, and the negative buckle is clamped to the negative slot; the negative buckle and the negative slot are configured to restrict the sensor device from coming out of the module support in the opposite direction of the implantation direction of the sensor device.

In some optional embodiments, the guide needle comprises a plastic handle and a puncture needle;
the sensor device comprises a sensor bottom shell, a sensor support, and a sensor; the sensor support is provided on the sensor bottom shell, and the sensor is clamped between the sensor support and the sensor bottom shell;
the guide needle passes through the sensor support and the sensor bottom shell, so that the puncture needle is located on the side of the sensor bottom shell facing away from the sensor support; the surface of the plastic handle facing the puncture needle is flush with the surface of the sensor bottom shell facing away from the sensor support.

In some optional embodiments, the sensor support is provided with a perforation, the plastic handle passes through the perforation, and a sealing rubber ring is provided between the plastic handle and the perforated inner wall of the sensor support.

In some optional embodiments, a sealing column is provided in the bottom shell, and the sealing column butts against the surface of the sensor bottom shell facing away from the sensor support;
an opening is provided at one end of the sealing column butting against the sensor bottom shell, and the puncture needle can be extended into the sealing column through the opening.

### BRIEF DESCRIPTION OF DRAWINGS

FIG. 1 is a schematic diagram of the overall structure of the sensor module in the embodiment of the present application;
FIG. 2 is a schematic diagram of an explosive structure of the sensor module in the embodiment of the present application;
FIG. 3 is a schematic diagram of the partial structure of the sensor module in the embodiment of the present application;
FIG. 4 is a structural diagram when the module support and the sensor device are separated in the embodiment of the present application;
FIG. 5 is a structural diagram of the sensor device fixed to the module support in the embodiment of the present application;
FIG. 6 is a structural diagram of another part of the sensor module in the embodiment of the present application;
FIG. 7 is a schematic diagram of a cross-sectional structure of the sensor module in the embodiment of the present application;
FIG. 8 is a schematic diagram of another cross-sectional structure of the sensor module in the embodiment of the present application;
FIG. 9 is a schematic diagram of the explosive structure of the sensor device in the embodiment of the present application;
FIG. 10 is a schematic diagram of a cross-sectional structure of the sensor device in the embodiment of the present application;
FIG. 11 is a schematic diagram of the explosive structure of the auxiliary implantation device in the embodiment of the present application;
FIG. 12 is a structural diagram when the lower cover and the outer shell are separated in the embodiment of the present application;
FIG. 13 is a schematic diagram of a cross-sectional structure of the auxiliary implantation device in the embodiment of the present application;
FIG. 14 is a schematic diagram of a cross-sectional structure when the auxiliary implantation device is assembled with the sensor module in the embodiment of the present application;
FIG. 15 is a schematic diagram of a cross-sectional structure of the main support sleeved in the inner sleeve in the embodiment of the present application;
FIG. 16 is a structural diagram of the process of assembling the auxiliary implantation device with the sensor module in the embodiment of the present application;
FIG. 17 is a structural diagram of the assembled auxiliary implantation device and the sensor module in the embodiment of the present application;
FIG. 18 is a structural diagram when the button module is assembled with the outer shell in the embodiment of the present application;
FIG. 19 is a structural diagram of a cross-sectional structure of the inner sleeve sleeved in the needle return support in the embodiment of the present application;
FIG. 20 is a schematic diagram of a cross-sectional structure of the clamping component clamping the guide needle in the embodiment of the present application;
FIG. 21 is a schematic diagram of another cross-sectional structure of the auxiliary implantation device in the embodiment of the present application;
FIG. 22 is a schematic diagram of another cross-sectional structure of the auxiliary implantation device in the embodiment of the present application;
FIG. 23 is a structural diagram of the packaging box open in the sensor module in the embodiment of the present application;
FIG. 24 is a structural diagram of removing the lower cover of the auxiliary implantation device in the embodiment of the present application;
FIG. 25 is a schematic diagram of a cross-sectional structure when the sensor device is implanted into the human body using an auxiliary implantation device in the embodiment of the present application;
FIG. 26 is a structural diagram when the auxiliary implantation device is picked up after FIG. 24;
FIG. 27 is a structural diagram of recycling the guide needle using the lower cover in the embodiment of the present application;
Fig. 28 is a structural diagram of the auxiliary implantation device in the embodiment of the present application when it is not in use.

Callouts:
100-sensor module; 110-packaging box; 111-bottom shell; 1111-clamping structure; 112-box cover; 120-module support; 121-positive buckle; 122-negative buckle; 123-elastic wall; 124-first support buckle; 125-second support buckle; 126-limiting groove; 127-snap-fit structure; 130-sensor device; 1301-positive slot; 1302-negative slot; 1303-perforation; 1304-first sealing rubber ring; 131-sensor bottom shell; 1311-glue tank; 1312-second sealing rubber ring; 132-sensor upper shell; 133-sensor; 134-sensor support; 135-circuit board; 136-application adhesive tape; 140-guide needle; 141-plastic handle; 142-needle body; 150-desiccant; 160-sealing column; 200-reusable auxiliary implantation device; 201-second limiting structure; 2011-second sleeve buckle; 2012-needle return buckle; 202-first limiting structure; 2021-overlapping part; 2022-first sleeve buckle; 210-needle return support; 211-clamping component; 2111-clamping rod; 220-inner sleeve; 221-top rib; 222-sleeve limiting rib; 230-main support; 231-second annular buckle; 232-support hook; 240-outer shell; 241-first annular buckle; 242-button hole; 243-limiting buckle; 244-button limiting table; 250-lower cover; 251-annular slot; 252-lower cover buckle; 260-button module; 261-button body; 262-button spring; 263-button limiting rib; 264-button mandril; 270-main spring; 280-needle return spring; 290-needle return mandril.

### DETAILED DESCRIPTION OF THE EMBODIMENTS

Technical solutions in the embodiments of the present application will be described clearly and completely in combination with figures in the embodiments of the invention. Obviously, the described embodiments are only part, but not all, of the embodiments of the invention. All other embodiments obtained by those of ordinary skill in the art without creative work based on the embodiments of the present application are within the scope of protection of the present application.

The reusable auxiliary implantation device in the embodiment of the present application can be configured to assist in implanting the sensor device into the user's skin. After the sensor device is implanted into the user's skin, the reusable auxiliary implantation device can also restore its initial state for future use, thereby achieving the reuse function of the reusable auxiliary implantation device.

Based on this, the solution in the embodiment of the present application may include two independent structures: a reusable auxiliary implantation device and a sensor module. When it is necessary to implant the sensor device into the user's skin, the sensor device in the sensor module can be assembled with the reusable auxiliary device. After the implantation is completed, the remaining structure on the reusable auxiliary implantation device can be removed to restore the initial state of the reusable auxiliary implantation device for the next use.

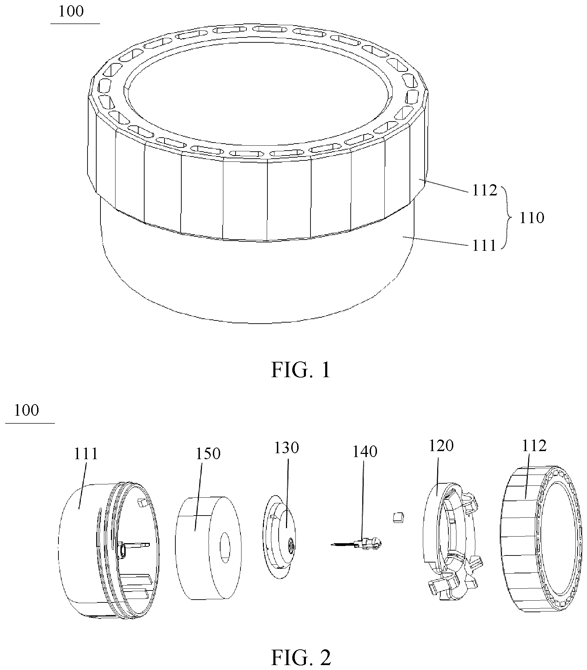

Therefore, referring to FIGS. 1 and 2, the sensor module 100 in the embodiment of the present application may comprise a packaging box 110, a module support 120, a sensor device 130, a guide needle 140, and a desiccant 150 arranged inside the packaging box 110. The packaging box 110 may comprise a bottom shell 111 and a box cover 112, with a detachable connection between the bottom shell 111 and the box cover 112, allowing the box cover 112 to be closed to the bottom shell 111 or removed from the bottom shell 111.

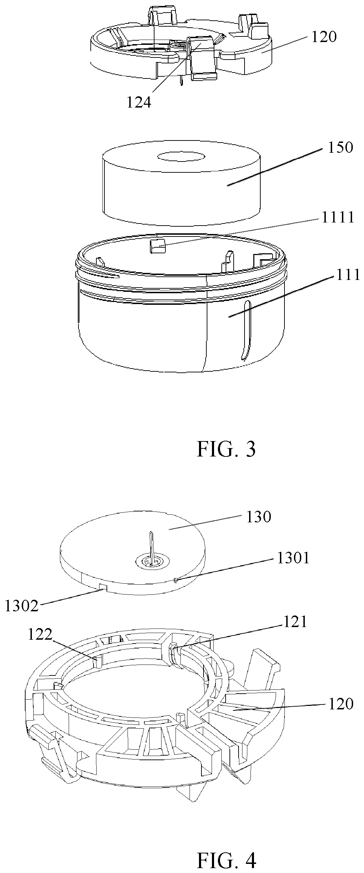

Referring to FIG. 3, there is a detachable connection between the module support 120 and the bottom shell 111. Specifically, the module support 120 can be installed along the circumference of the bottom shell 111 on the inner wall of the bottom shell 111. The inner wall of the bottom shell 111 can be provided with a clamping structure 1111, and the module support 120 can be relatively fixed to the inner wall of the bottom shell 111 through the clamping structure 1111. When external force is applied to the clamping structure 1111, it can also cause the module support 120 to detach from the clamping structure 1111, making it easier to remove the module support 120 from the bottom shell 111.

Referring to FIG. 4, there is an annular hole in the middle of the module support 120, and the annular hole can be used to accommodate the sensor device 130, so that when the sensor device 130 is installed on the module support 120, the inner wall of the annular hole is in contact with the side wall of the sensor device 130, thereby playing a preliminary limiting role for the sensor device 130.

The inner wall of the annular hole can also be provided with a positive buckle 121 and a negative buckle 122, respectively. Correspondingly, the sensor device 130 can be provided with a positive slot 1301 and a negative slot 1302, respectively. When the sensor device 130 is placed in the annular hole, the positive buckle 121 can be clamped in the positive slot 1301, and the negative buckle 122 can be clamped in the negative slot 1302. At this time, under the joint action of the positive buckle 121 and the negative buckle 122, the sensor device 130 and the module support 120 can be relatively fixed.

Specifically, the positive slot 1301 of the sensor device 130 is provided on the side of the sensor device 130, and a part of the positive slot 1301 extends to the bottom of the sensor device 130. When the positive buckle 121 is clamped in the slot, a part of the positive buckle 121 is pressed against the bottom surface of the sensor device 130.

The negative slot 1302 of the sensor device 130 is provided on the side of the sensor device 130, and the opening of the negative slot 1302 is opened on the side facing the top of the sensor device 130. When the negative buckle 122 is clamped in the negative slot 1302, the negative buckle 122 can be pressed against the bottom of the negative slot 1302.

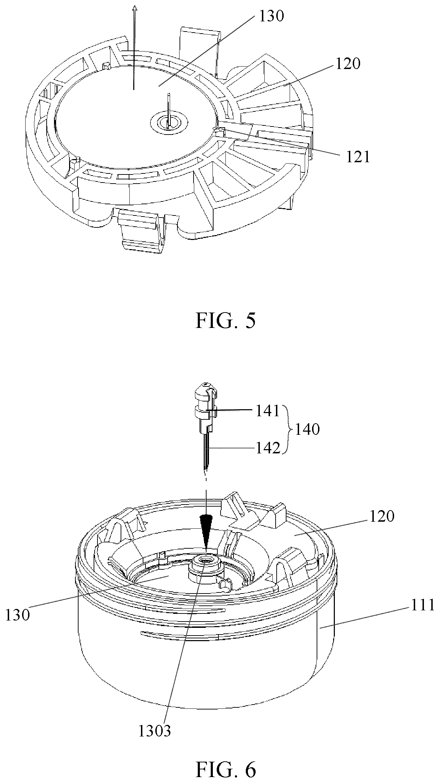

Referring to FIGS. 4 and 5 together, when a force is applied to the sensor device 130 along the axis direction of the annular hole, if the force acts on the top of the sensor device 130 (as indicated by the arrow in FIG. 5), it means that the sensor device 130 is pressed from top to bottom. At this point, the force acting on the sensor device 130 can be understood as the driving force for implanting the sensor device 130 into the user's skin, and the driving force is used to drive the sensor device 130 to move in the implantation direction. The positive buckle 121 can be an elastic structure. When the sensor device 130 is subjected to a top-down force, the force can drive the sensor device 130 to move downwards, causing the positive buckle 121 to detach from the positive slot 1301. During this process, the bottom of the negative slot 1302 gradually moves away from the negative buckle 122, so the negative slot 1302 and the negative buckle 122 will not affect the sensor device 130 detaching from the module support 120.

On the contrary, when the sensor device 130 is pushed from bottom to top, the force acting on the sensor device 130 can be understood as driving the sensor device 130 to move along the opposite direction of the implantation direction. Due to the negative buckle 122 being pressed against the bottom of the negative slot 1302, when this force is applied to the sensor device 130, the sensor device 130 cannot move relative to the module support 120 under the snap-fit action of the negative slot 1302 and the negative locking buckle 122. In other words, the negative slot 1302 and the negative buckle 122 can be used to limit the reverse detachment of the sensor device 130 from the module support 120 along its implantation direction.

Referring to FIG. 6, the sensor device 130 may be provided with a perforation 1303 for passing through the guide needle 140, and the guide needle includes a plastic handle 141 and a needle body 142. The needle body 142 can be located in the needle slot of the puncture needle after passing through the perforation 1303. When implanting the sensor device 130 into the user's skin, the guide needle 140 can pierce the skin before the puncture needle, thereby guiding the puncture of the puncture needle.

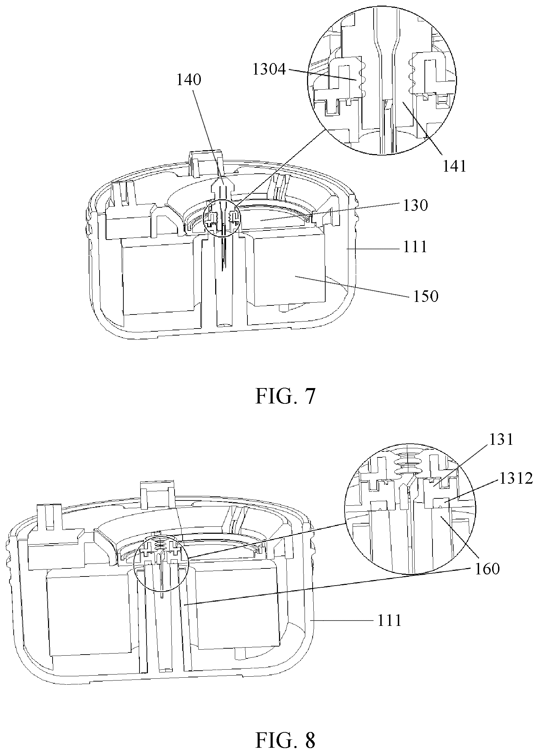

Referring to FIG. 7, the inner wall of the perforation 1303 may be provided with a first sealing rubber ring 1304. When the guide needle 140 is installed on the sensor device 130 through the perforation 1303, a part of the plastic handle 141 is located inside the perforation 1303, and the plastic handle 141 and the first sealing rubber ring 1304 are pressed against each other, thereby ensuring the sealing between the guide needle 140 and the sensor device 130.

In addition, referring to FIG. 8, a sealing column 160 can also be provided at the bottom of the bottom shell 111, and the sealing column 160 extends along the axial direction of the bottom shell 111. The end of the sealing column 160 facing away from the bottom of the bottom shell 111 has an opening. When the module support 120 with the sensor device 130 is installed on the bottom shell 111, the puncture needle of the sensor device 130 and the needle body 142 of the guide needle 140 can both extend into the sealing column 160 through the opening, thereby improving the sealing performance between the bottom shell 111 and the sensor device 130.

As shown in FIG. 8, a second sealing rubber ring 1312 can also be provided between the bottom of the sensor device 130 and the top of the sealing column 160, thereby further improving the sealing performance between the bottom shell 111 and the sensor device 130. The desiccant 150 can be placed inside the bottom shell 111 around the sealing column 160 to maintain dry inside the bottom shell 111.

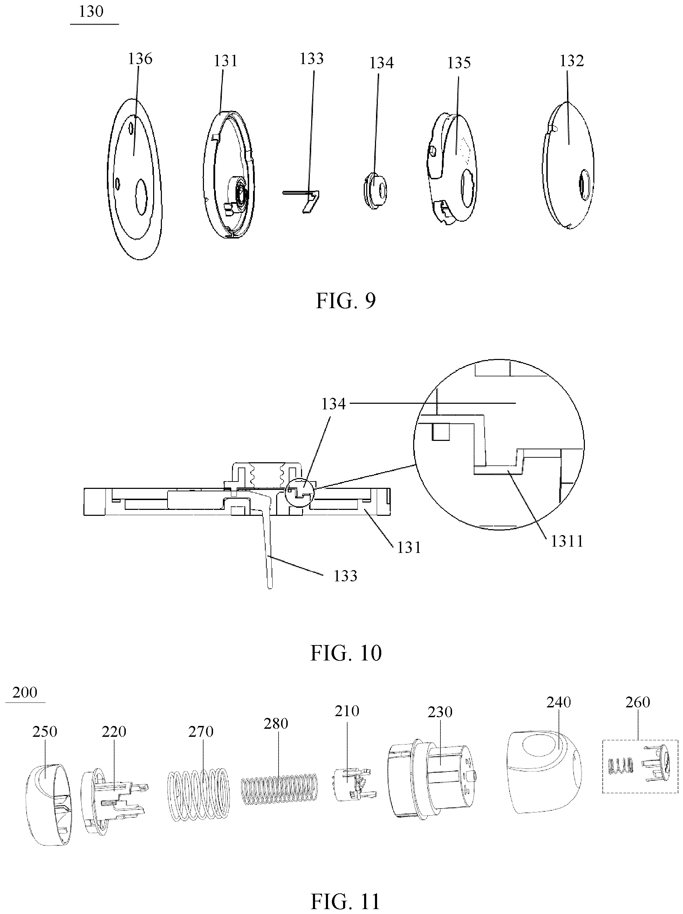

Referring to FIG. 9, the sensor device 130 may comprise a sensor bottom shell 131, a sensor upper shell 132, a sensor 133, a sensor support 134, a circuit board 135, and an application adhesive tape 136, wherein the sensor 133, the sensor support 134, and the circuit board 135 are all disposed on the sensor bottom shell 131.

Specifically, referring to FIGS. 9 and 10, the sensor 133 can be fixedly connected to the sensor bottom shell 131. The sensor 133 is configured for a puncture needle piercing into the user's skin to pass through the sensor bottom shell 131, so that the puncture needle is located outside of the sensor bottom shell 131. The sensor support 134 is located on the side of the sensor 133 away from the sensor bottom shell 131, so that the sensor support 134 can press the sensor 133 tightly onto the sensor bottom shell 131. The sensor bottom shell 131 can be provided with a glue tank 1311; a part of the sensor support 134 can be located inside the glue tank 1311, and the sensor support 134 can be bonded to the glue tank 1311 through glue, thereby achieving the fixation between the sensor support 134 and the sensor bottom shell 131.

The circuit board 135 is provided with an avoidance hole. When the circuit board 135 is fixed in the sensor bottom shell 131, the sensor support 134 is located inside the avoidance hole, so that the bottom of the circuit board 135 can be fixedly connected to the sensor bottom shell 131.

The sensor upper shell 132 is located on the side of the circuit board 135 facing away from the sensor bottom shell 131. The connector on the circuit board 135 can make contact with the contacts of the sensor 133 to achieve the connection of the signal transmission channels. The sensor upper shell 132 can be closed to the sensor bottom shell 131, thereby ensuring that a relatively closed space is formed inside the sensor bottom shell 131, avoiding contamination of the sensor 133 by external substances.

The application adhesive tape 136 can be fixed to the side of the sensor bottom shell 131 facing away from the sensor upper shell 132. When the sensor device 130 is implanted into the user's skin, the application adhesive tape 136 can be used to adhere the sensor device 130 to the surface of the user's skin, thereby enhancing the firmness of the connection between the sensor 133 and the user's skin.

As mentioned earlier, the sensor device 130 has a perforation 1303 that passes through the guide needle 140. Based on this, the application adhesive tape 136, sensor support 134, sensor bottom shell 131, and sensor upper shell 132 can all be provided with perforations 1303, so that the guide needle 140 can pass through the perforation 1303 of the sensor upper shell 132 to the perforation 1303 of the application adhesive tape 136. At this point, the first sealing rubber ring 1304 can be provided on the inner wall of the perforation 1303 of the sensor support 134, and the inner wall of the perforation 1303 provided on the sensor bottom shell 131 can also be provided with a protrusion. Correspondingly, the plastic handle 141 of the guide needle 140 can also be provided with a limiting groove, and the protrusion can be clamped in the limiting groove 126 to prevent the guide needle 140 from rotating relative to the sensor device 130.

In addition, when the plastic handle 141 of the guide needle 140 is located in the perforation 1303, the surface of the plastic handle 141 facing the puncture needle 142 can also be flush with the surface of the sensor bottom shell 131 facing away from the sensor support 134. That is to say, the plastic handle 141 will not protrude from the surface of the sensor bottom shell 131. When using the guide needle 140 to assist the sensor device 130 in piercing the user's skin and then withdrawing the guide needle 140 from the sensor device 130, the guide needle 140 can be directly pulled out of the sensor device 130 at once. If the plastic handle 141 protrudes from the surface of the sensor bottom shell 131, when withdrawing the needle, it is necessary to first fully withdraw the plastic handle 141 into the perforation 1303, and then pull out the guide needle 140 from the sensor device 130. Thus, the design in this embodiment can make it more convenient and simpler to remove the guide needle 140 from the sensor device 130.

The reusable implantation device in the embodiment of the present application may comprise an injection structure and a reset structure, wherein the module support 120 installed with the sensor device 130 can be connected to the injection structure, and the injection structure can move along the injection direction to facilitate the implantation of the sensor device 130 into the human body. Subsequently, when the injection structure moves in the opposite direction of the injection direction, the injection structure can also detach from the sensor device 130 with the module support 120 and guide needle 140, allowing the sensor device 130 to stay in the human body. Finally, the reset structure can drive the module support to separate from the injection structure, and drive the guide needle to detach from the injection structure, thereby enabling the reusable implantation device to restore its initial state.

Based on this, referring to FIG. 11, the above injection structure may comprise a needle return support 210 and an inner sleeve 220. In addition, the reusable auxiliary implantation device 200 in the embodiment of the present application may further comprise a main support 230, an outer shell 240, a lower cover 250, and a button module 260, wherein the inner sleeve 220 is sleeved and connected to the needle return support 210, and the main support 230 is sleeved and connected to the inner sleeve 220; the outer shell 240 is sleeved and connected to the main support 230, and the lower cover 250 can be closed to the outer shell 240; the button module 260 is located on top of the outer shell 240.

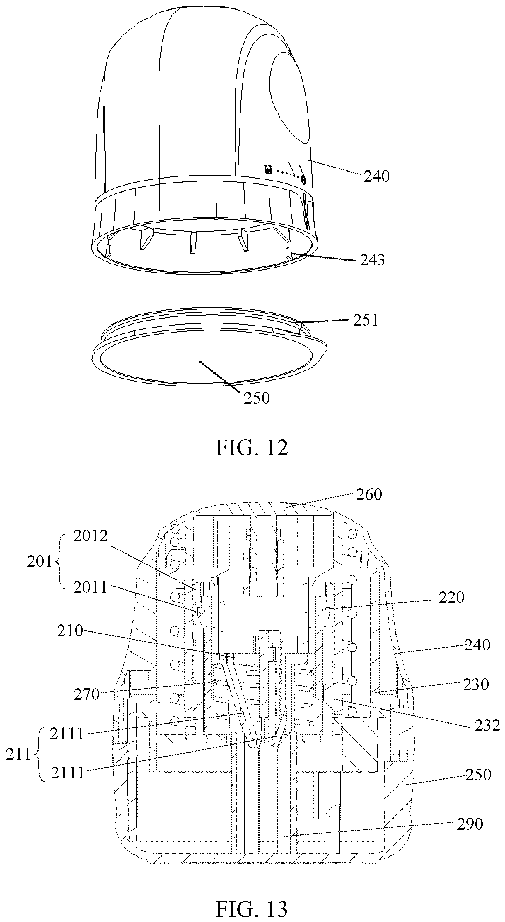

Referring to FIG. 12, there is a detachable connection between the lower cover 250 and the outer shell 240. When the lower cover 250 is closed to the outer shell 240, the reusable auxiliary implantation device 200 is in an unused state. When the reusable auxiliary implantation device 200 is required, the lower cover 250 can be removed from the outer shell 240, exposing the inner sleeve 220 at the bottom of the outer shell 240, so that the inner sleeve 220 can be connected to the sensor module 100.

For example, as shown in FIG. 12, the inner wall of the outer shell 240 may be provided with a limiting buckle 243, and the inner wall of the lower cover 250 may be provided with an annular slot 251, which is arranged around the circumference of the lower cover 250. When the lower cover 250 is closed to the outer shell 240, the limiting buckle 243 is clamped in the annular slot 251. It can be understood that by provision of the annular slot 251, regardless of the angle at which the lower cover 250 is closed to the outer shell 240, the limiting buckle 243 can be quickly engaged with the annular slot 251, without alignment. When it is necessary to remove the lower cover 250, the lower cover 250 is only pulled down so that the limiting buckle 243 is disengaged from the limiting slot.

In this embodiment, the inner sleeve 220 is provided with a first engagement structure, and the module support 120 is provided with a second engagement structure. When installing the sensor module 100 with the inner sleeve 220, the first engagement structure can be engaged with the second engagement structure, thereby fixing the module support 120 relative to the inner sleeve. When the first engagement structure is detached from the second engagement structure, the inner sleeve 220 can detach from the module support 120.

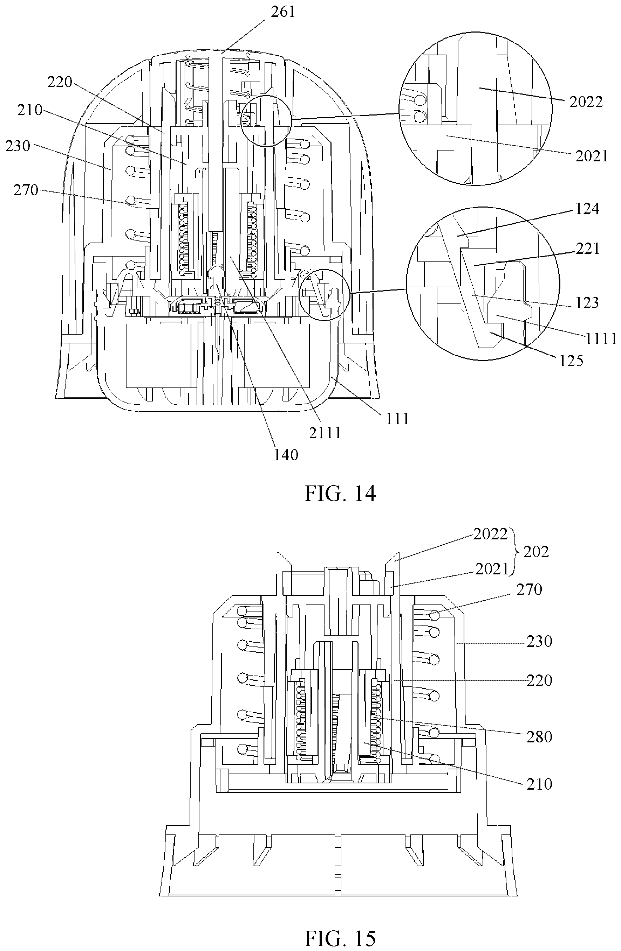

Referring to FIG. 14, the first engagement structure may comprise a top rib 221 arranged on the inner wall of the inner sleeve 220, and the second engagement structure may comprise a first support buckle 124 arranged on the module support 120, wherein the top rib 221 is arranged near the bottom of the inner sleeve 220. When the first engagement structure is engaged with the second engagement structure, the first support buckle 124 is pressed against the top rib 221. If there are at least two top ribs 221 provided on the inner sleeve 220, correspondingly, there are also at least two first support buckles 124. When each top rib 221 is pressed against the corresponding first support buckle 124, the inner sleeve 220 and the module support 120 can be kept relatively fixed.

Based on this, looking back to FIG. 1, the module support 120 is also provided with a second support buckle 125. When the module support 120 is placed inside the bottom shell 111, the second support buckle 125 can be pressed against the clamping structure 1111, thereby achieving relative fixation between the module support 120 and the bottom shell 111.

Furthermore, referring to FIG. 14 again, the module support 120 is also provided with an elastic wall 123. The first support buckle 124 and the second support buckle 125 are both connected to the elastic wall 123, and the second support buckle 125 is located below the first support buckle 124.

The elastic wall 123 can be inclined towards the axis direction of the module support 120. Along the radial direction of the inner sleeve 220, the distance between the second support buckle 125 and the axis of the inner sleeve 220 is greater than the distance between the first support buckle 124 and the axis of the inner sleeve 220.

Before assembling the module support 120 with the inner sleeve 220, the module support 120 is pressed against the bottom shell 111 through the second support buckle 125 and the clamping structure 1111. When assembling the bottom shell 111 from bottom to top with the inner sleeve 220, the top rib 221 can press the elastic wall 123 inwards, causing the second support buckle 125 to detach from the clamping structure 1111. During this process, the top rib 221 can be pressed under the first support buckle 124 to complete the fixation between the inner sleeve 220 and the module support 120, and the bottom shell 111 can be separated from the module support 120.

Referring to FIGS. 13 and 14 together, the needle return support 210 is provided with a clamping component 211, and the clamping component has a clamped state and a non-clamped state. When the clamping component 211 is in the clamped state, the clamping component 211 can be configured to clamp the guide needle 140. When the clamping component 211 is in the non-clamped state, the guide needle 140 can be released from the clamping component 211. Specifically, when the module support 120 with the sensor device 130 installed is relatively fixed to the inner sleeve 220, the clamping component 211 can be clamped to the plastic handle 141 of the guide needle 140. When the sensor device 130 is not implanted into the user's skin, the clamping component 211 and the guide needle 140 remain relatively fixed.

Referring to FIGS. 14 and 15, a first limiting structure 202 is provided between the inner sleeve 220 and the main support 230. When the first limiting structure 202 is in a limiting state, the inner sleeve 220 and the main support 230 are relatively fixed. When the first limiting structure 202 is in an unlocked state, the inner sleeve 220 can move along the axis direction of the inner sleeve 220 relative to the main support 230.

The first limiting structure 202 may comprise a first sleeve buckle 2022 disposed on the inner wall of the inner sleeve 220 and an overlapping part 2021 disposed inside the main support 230, wherein the first sleeve buckle 2022 protrudes from the inner wall surface of the inner sleeve 220, and the overlapping part 2021 is located in the middle of the main support 230. There is a certain gap between the overlapping part 2021 and the wall of the main support 230, which can be used to pass through the side wall of the inner sleeve 220. That is to say, the inner sleeve 220 is sleeved on the overlapping part 2021, and the main support 230 is sleeved on the inner sleeve 220.

When the first limiting structure 202 is in a limiting state, that is, the first sleeve buckle 2022 is pressed against the top surface of the overlapping part 2021. When the first limiting structure 202 is in an unlocked state, that is, the first sleeve buckle 2022 is detached from the overlapping part 2021. In addition, there is a main spring 270 connected between the inner sleeve 220 and the main support 230. When the first limiting structure 202 is in a limiting state, the main spring 270 is in a compressed state.

When the first limiting structure 202 switches to the unlocked state, the first sleeve buckle 2022 disengages from the overlapping part 2021. Due to the elastic potential energy of the main spring 270, the main spring 270 can drive the inner sleeve 220 to move downwards relative to the main support 230.

It is worth noting that when assembling the sensor module 100 with the reusable auxiliary implantation device 200, the box cover 112 of the sensor module 100 can be removed first, and then the bottom shell 111 can be assembled with the reusable auxiliary implantation device 200 to ensure that the sensor device 130 is always in a sealed state before being implanted into the user's skin.

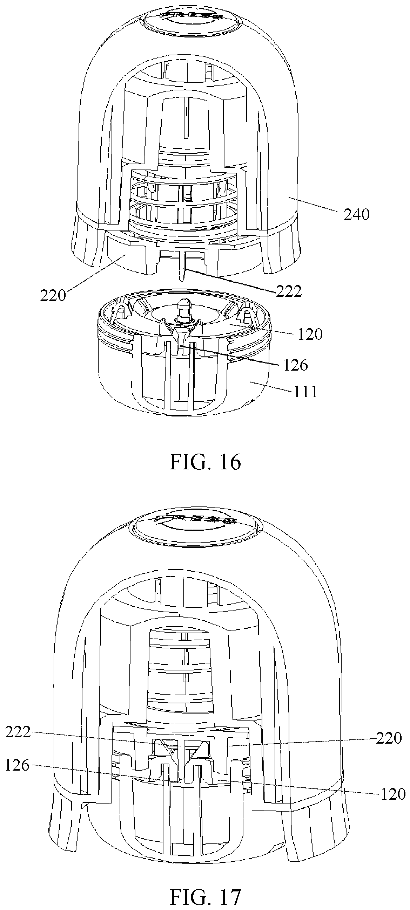

On this basis, referring to FIGS. 16 and 17, the bottom of the inner sleeve 220 can also be provided with a sleeve limiting rib 222. Correspondingly, the module support 120 can be provided with a limit groove 126. When assembling the bottom shell 111 from the bottom of the outer shell 240 with the inner sleeve 220, the limiting rib can be inserted into the limiting groove 126 to ensure alignment between the top rib 221 and the first support buckle 124.

In order to facilitate the accurate insertion of the sleeve limiting rib 222 into the limiting groove 126, an alignment line can also be provided on the surface of the outer shell 240, and an alignment line can also be provided on the surface of the bottom shell 111. When the two alignment lines are aligned, it means that the sleeve limiting rib 222 is aligned with the limiting groove 126.

After fixing the module support 120 and the inner sleeve 220, the bottom shell 111 can also be pulled down to separate the module support 120 from the bottom shell 111, making it easier for the subsequent implantation of the sensor device 130.

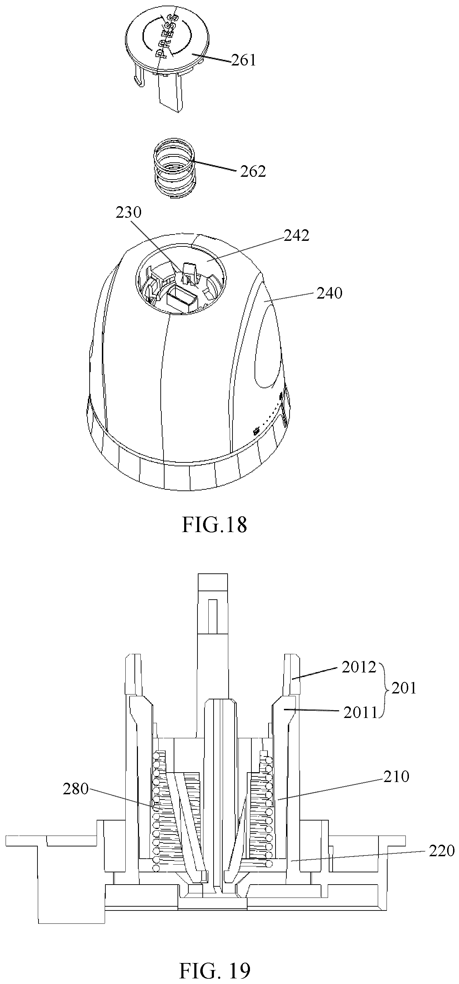

Referring to FIG. 18, the button module 260 may comprise a button body 261 and a button spring 262. The top of the outer shell 240 may have a button hole 242, and the button body 261 may pass through the button hole 242. The button spring 262 may be connected between the button body 261 and the main support 230. Under the external force, the button body 261 can move relative to the outer shell 240 along the axis direction of the inner sleeve 220. During this process, the button body 261 can compress the button spring 262. When the external force disappears, the button spring 262 can drive the button body 261 to restore to its initial position.

Combining FIG. 14 and FIG. 18, when the first limiting structure 202 is in a limiting state, that is, the first sleeve buckle 2022 is pressed against the top surface of the overlapping part 2021. At this time, when the button body 261 is pressed down, the bottom of the button body 261 can contact the top of the first inner cylinder buckle. During the continuous pressing of the button body 261, the button body 261 can press the first sleeve buckle 2022, causing the first sleeve buckle 2022 to detach from the top surface of the overlapping part 2021, thereby completing the switching of the first limiting structure 202 from the limiting state to the unlocked state.

The bottom of the button body 261 may be provided with a first guide surface, which is a sloping surface, and the top of the first sleeve buckle 2022 may be provided with a second guide surface, which is also a sloping surface. Moreover, the first guide surface and the second guide surface are arranged parallel to each other. When the button body 261 comes into contact with the first sleeve buckle 2022, the first guide surface comes into contact with the second guide surface. As the button body 261 continues to move downwards, the first guide surface can exert an outward force on the second guide surface to press the first sleeve buckle 2022, causing the first sleeve buckle 2022, which was originally facing the overlapping part 2021, to be pressed into a misaligned state with the overlapping part 2021, so that the first sleeve buckle 2022 can move to the gap between the overlapping part 2021 and the inner wall of the main support 230.

It is worth noting that in the initial state of the reusable auxiliary implantation device 200 in this embodiment, the first limiting structure 202 is in an unlocked state, and the inner sleeve 220 and the main support 230 can move relatively. The first sleeve buckle 2022 of the inner sleeve 220 is located below the overlapping part 2021.

Referring to FIG. 19, a second limiting structure 201 can be provided between the needle return support 210 and the inner sleeve 220. When the second limiting structure 201 is in a limiting state, the needle return support 210 is relatively fixed to the inner sleeve 220. When the second limiting structure 201 is in an unlocked state, the needle return support 210 can move relative to the inner sleeve 220 along the axis direction of the inner sleeve 220.

Specifically, the second limiting structure 201 may comprise a needle return buckle 2012 disposed on the outer wall of the needle return support 210 and a second sleeve buckle 2011 disposed on the inner sleeve 220. When the second limiting structure 201 is in a limiting state, the needle return buckle 2012 is pressed against the bottom of the second sleeve buckle 2011, thereby fixing the needle return support 210 relative to the inner sleeve 220.

A needle return spring 280 can also be connected between the needle return support 210 and the inner sleeve 220. When the second limiting structure 201 is in the limiting state, the needle return spring 280 is in a compressed state. When the second limiting structure 201 switches to the unlocked state, the needle return spring 280 has elastic potential energy, which can drive the needle return support 210 to move upwards relative to the inner sleeve 220.

Referring to FIGS. 13 and 19 together, the inner wall of the main support 230 is also provided with a third limiting structure, which can be located near the bottom of the main support 230. As mentioned earlier, the inner sleeve 220 can move downwards relative to the main support 230, and the third limiting structure can be configured to limit the movement distance of the inner sleeve 220 relative to the main support 230, preventing the inner sleeve 220 from moving too far and causing plastic deformation of the main spring 270.

The third limiting structure may comprise support hooks 232 arranged on the inner wall of the main support 230, and the number of support hooks 232 may be the same as the number of second sleeve buckles 2011. Moreover, they are arranged in one-to-one correspondence with each other. When the inner sleeve 220 moves relative to the main support 230, the second limiting structure 201 is in a limiting state, that is, the inner sleeve 220 is relatively fixed to the needle return support 210, and the inner sleeve 220 drives the needle return support 210 to move downwards together. Due to the fact that the needle return buckle 2012 is pressed against the bottom of the second sleeve buckle 2011, when the support hook 232 and the inner sleeve 220 play a limiting role, the needle return buckle 2012 is sandwiched between the support hook 232 and the second sleeve buckle 2011. At this point, if the inner sleeve 220 continues to move downwards, the support hook 232 and the second sleeve buckle 2011 can cooperate to press the needle return buckle 2012, causing the needle return buckle 2012 to disengage from the contact with the second sleeve buckle 2011, thereby causing the support hook 232 to press against the bottom of the second sleeve, so as to relatively fix the main support 230 and the inner sleeve 220.

It can be understood that the support buckle in this embodiment cannot only be used to limit the movement distance of the inner sleeve 220, but also to drive the second limiting structure 201 to switch from the limiting state to the unlocked state. Moreover, the continuous downward movement of the inner sleeve 220 relative to the main support 230 is the process of implanting the sensor device 130 into the user's skin. When the support hook 232 is pressed against the second sleeve buckle 2011, it represents that the sensor device 130 is implanted in place.

The bottom of the needle return buckle 2012 can also be provided with a third guide surface, and the top of the support hook 232 can also be provided with a fourth guide surface, both of which are inclined towards the axis direction of the inner sleeve 220. Moreover, the third guide surface is parallel to the fourth guide surface. When the needle return buckle 2012 comes into contact with the support hook 232, the third guide surface fits with the fourth guide surface. Under the pressure of the second sleeve buckle 2011, the third guide surface can slide along the fourth guide surface, making it easier for the needle return buckle 2012 to detach from the second sleeve buckle 2011.

Due to the clamping component 211 provided on the needle return support 210 for clamping the guide needle 140, after the sensor device 130 is implanted into the user's skin, the elastic potential energy of the needle return spring 280 can drive the needle return support 210 to move upwards relative to the inner sleeve 220, as the second limiting structure 201 is in an unlocked state. During this process, the needle return support 210 can drive the guide needle 140 to move upwards, thereby causing the guide needle 140 to detach from the sensor device 130.

Referring again to FIGS. 13 and 14, the clamping component 211 may comprise two clamping rods 2111, which can be symmetrically arranged on the inner wall of the needle return support 210 with the axis of the needle return support 210 as the axis of symmetry. The clamping rod 2111 is rotatably connected to the inner wall of the needle return support 210 relative to the needle return support 210. Specifically, one end of the clamping rod 2111 is connected to the needle return support 210, and the other end is a free end. When the clamping component 211 is in a clamped state, the free ends of the two clamping rods 2111 are close to each other, thereby clamping the guide needle 140.

The clamping rod 2111 can be, for example, an elastic rod that is inclined to the axis of the needle return support 210. When two clamping rods 2111 are pressed by external force, causing the free ends of the two clamping rods 2111 to move away from each other, the distance between the free ends of the two clamping ends increases, making it impossible to clamp the guide needle 140. The guide needle 140 can naturally fall off under the action of gravity. After the external force disappears, the clamping rod 2111 can return to the clamped state under the action of elasticity.

Alternatively, the clamping rod 2111 and the inner wall of the needle return support 210 may be provided with a reset member. In the initial state, under the action of the reset member, the free ends of the two clamping rods 2111 are close to each other, so that the clamping component 211 is in a clamped state. When the clamping rod 2111 is driven to rotate relative to the needle return support 210 by external force, causing the free ends of the two clamping rods 2111 to move away from each other, the guide needle 140 can be detached. At this point, the external force disappears, and under the action of the reset component, the clamping rod 2111 can rotate relative to the needle return support 210, thereby returning to the clamped state.

It can be understood that when the module support 120 is fixed relative to the inner sleeve 220, the plastic handle 141 of the guide needle 140 protrudes from the module support 120 and extends towards the button module 260. During the process of fixing the module support 120 and the inner sleeve 220, the guide needle 140 can press the two clamping rods 2111 from bottom to top, causing the free ends of the two clamping rods 2111 to move away from each other, so that the plastic handle 141 of the guide needle 140 can pass between the free ends of the two clamping rods 2111. After the module support 120 is fixed, the clamping rod 2111 can clamp the plastic handle 141 of the guide needle 140 under the reset action and maintain relative fixation with the plastic handle 141.

After the needle return support 210 moves upwards relative to the inner sleeve 220 and drives the guide needle 140 to detach from the sensor device 130, the reusable auxiliary implantation device 200 can also be configured to drive the clamping component 211 to switch from a clamped state to a non-clamped state, so that the guide needle 140 can detach.

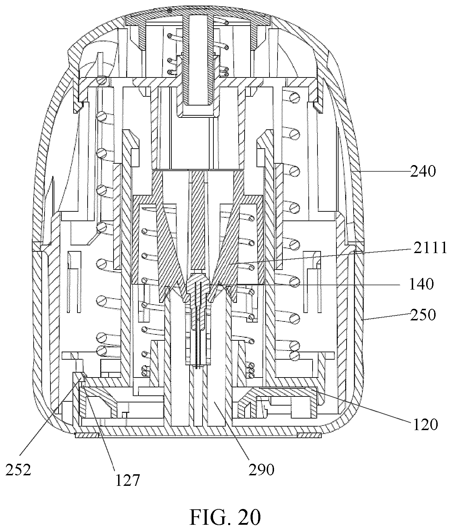

Based on this, in some embodiments, referring to FIG. 20, the reset structure may comprise a needle return mandril 290 disposed inside the lower cover 250, which extends along the axis direction of the lower cover 250 to protrude from the upper surface of the lower cover 250.

There may be two needle return mandrils 290, which are arranged in one-to-one correspondence with two clamping rods 2111. After the implantation of the sensor device 130 is completed, when assembling the lower cover 250 with the outer shell 240, the two needle return mandrils 290 can respectively act on the bottom of the two clamping rods 2111, and press the two clamping rods 2111 separately, so that the ends of the two clamping rods 2111 are far away. At this point, the guide needle 140 loses the clamping effect of the two clamping rods 2111 and can detach between the two clamping rods 2111 under the action of gravity.

The two needle return needle mandrils 290 are spaced apart, and the reset structure may also comprise a magnet located between the two return needle mandrils 290, in order to guide the guide needle 140 through magnetic attraction, so that the guide needle 140 can fall between the two return needle mandrils 290.

In addition, the reset structure may also comprise a lower cover buckle 252 located inside the lower cover 250, and correspondingly, the module support 120 is provided with a snap-fit structure 127. When assembling the lower cover 250 with the outer shell 240, the lower cover buckle 152 can also overlap with the upper surface of the buckle structure 127 and be pressed against the snap-fit structure 127. At this point, the module support 120 is not only fixed relative to the inner sleeve 220, but also fixed relative to the lower cover 250.

Referring to FIGS. 14 and 20 together, when separating the lower cover 250 from the outer shell 240 again, during the process of pulling down the lower cover 250, the lower cover buckle 152 presses down the snap-fit structure 127, causing the top rib 221 to separate from the first support buckle 124, and the module support 120 to detach from the inner sleeve 220. At this point, the guide needle 140 falls into the lower cover 250, and the module support 120 also falls into the lower cover 250, thereby achieving the recycling of the module support 120 and the guide needle 140.

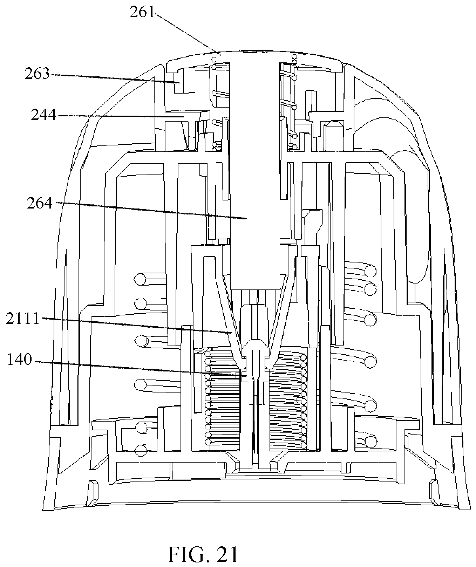

Alternatively, in some embodiments, referring to FIG. 21, there are first and second gears between the button module 260 and the outer shell 240. When the button module 260 is in the first gear with the outer shell 240, the button module 260 can be configured to drive the first limiting structure 202 to switch from the limiting state to the unlocked state. When the button module 260 is in the second gear with the outer shell 240, the button module 260 can be configured to drive the clamping component 211 to switch from the clamped state to the non-clamped state.

In specific implementation, the button module 260 may also comprise a button limiting rib 263, which is provided on the button body 261. The inner wall of the outer shell 240 can be provided with a button limiting table 244. When the button module 260 is in the first gear with the outer shell 240, the button limiting table 244 is directly facing the button limiting rib 263, and is located below the button limiting rib 263. When the button body 261 is pressed down, the distance it moves is limited to the distance between the button limiting rib 263 and the button limiting table 244, that is, the movable distance of the button body 261 is relatively short.

The outer shell 240 can also rotate around the axis of the main support 230 relative to the main support 230. When the outer shell 240 rotates, the relative position between the button limiting table 244 and the button limiting rib 263 can be changed. When the outer shell 240 rotates to the second gear with the button module 260, the circumferential direction of the outer shell 240 is misaligned between the button limiting table 244 and the button limiting rib 263. That is, during the downward movement of the button body 261, the button limiting table 244 will not come into contact with the button limiting rib 263.

Based on this, the button module 260 may also comprise a button mandril 264, which is connected to the button body 261 and extends along the axis direction of the inner sleeve 220. When the outer shell 240 is in the second gear with the button module 260, the downward movement distance of the button body 261 is not limited by the button limiting table 244, and the button body 261 can move downwards a longer distance. During this process, the button mandril 264 can pass through the overlapping part 2021 and extend between the two clamping rods 2111. After the button mandril 264 comes into contact with the clamping rod 2111, during the continuous downward movement of the button body 261, the button mandril 264 can press the clamping rod 2111, causing the clamping rod 2111 to rotate relative to the needle return support 210, thereby switching the clamping component 211 from a clamped state to a non-clamped state, so that the guide needle 140 can fall off.

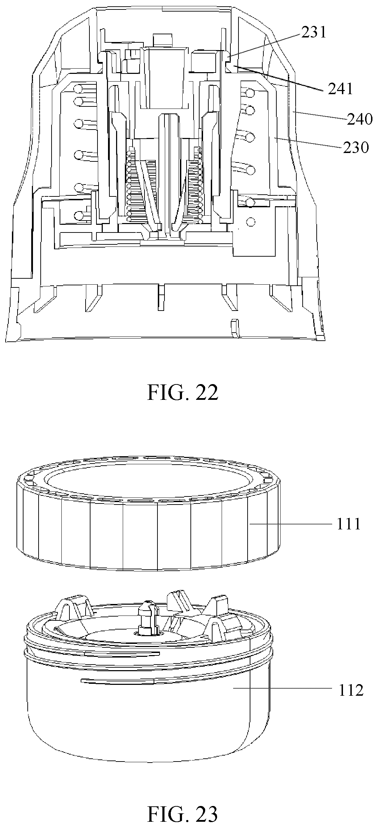

To facilitate the relative fixation of the outer shell 240 and the main support 230 in the direction perpendicular to the axis direction of the main support 230, as shown in FIG. 22, the inner wall of the outer shell 240 may be provided with a first annular buckle 241, and the outer wall of the main support 230 may be provided with a second annular buckle 231. The first annular buckle 231 can be pressed under the second annular buckle 241, so that the outer shell 240 and the main support 230 can be relatively fixed. When the outer shell 240 rotates relative to the main support 230, the first annular buckle 241 and the second annular buckle 231 always maintain contact to ensure that the outer shell 240 does not fall off from the main support 230.

In addition, two indicator marks can be provided on the surface of the outer shell 240, one of which is used to indicate that the outer shell 240 is in the first gear with the button support, and the other one is used to indicate that the outer shell 240 is in the second gear with the button support. In this way, when manually rotating the outer shell 240, the indicator mark can be used to ensure that the outer shell 240 rotates in place.

It is worth noting that when the button mandril 264 is provided, the button mandril 264 is used to recycle the guide needle 140. On this basis, when separating the module support 120 from the inner sleeve 220, the elastic wall 123 can also be manually pressed inwards to separate the top rib 221 from the first support buckle 124, thereby achieving the separation of the module support 120 from the inner sleeve 220. Alternatively, a lower cover buckle 252 can be installed inside the lower cover 250, and the lower cover buckle 252 can be used to engage with the snap-fit structure 127 of the module support 120 to achieve the recycling of the module support 120.

Based on the specific structures of the reusable auxiliary implantation device 200 and the sensor module 100 in the above embodiments, when using the reusable auxiliary implantation device 200 to implant the sensor device 130 provided in the sensor module 100 into the user's skin, the specific steps can be referred to below.

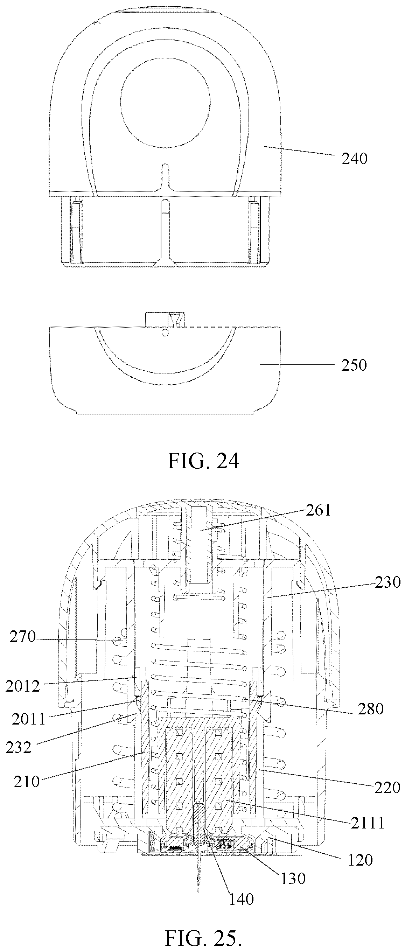

Firstly, as shown in FIGS. 23 and 24, the box cover 112 of the sensor module 100 is opened and the lower cover 250 of the reusable auxiliary implantation device 200 is removed.

Next, as shown in FIGS. 16 and 17, the reusable auxiliary implantation device 200 with the lower cover 250 removed is assembled from top to bottom with the bottom shell 111 of the sensor module 100. During this process, the sleeve limiting rib 222 of the inner sleeve 220 can be embedded into the upper limiting groove 126 of the module support 120 to achieve positioning.

In this state, the inner sleeve 220 and the main support 230 can move relatively. Therefore, after the sleeve limiting rib 222 is embedded in the limiting groove 126, the outer shell 240 can be pressed downwards to drive the main support 230 to move downwards together and compress the main spring 270. After the main spring 270 is compressed, it can drive the inner sleeve 220 to move downwards, so that the top rib 221 of the inner sleeve 220 presses the first support buckle 124 on the module support 120, and the first support buckle 124 disengages from the clamping structure 1111 of the bottom shell 111. At the same time, the top rib 221 is pressed against the first support buckle 124 to achieve the fixation of the module support 120 and the inner sleeve 220. Moreover, during this process, due to the relative fixation between the needle return support 210 and the inner sleeve 220, when the module support 120 is fixed to the inner sleeve 220, the clamping component 211 on the needle return support 210 clamps the guide needle 140.

As shown in FIG. 14, after the module support 120 is fixed to the inner sleeve 220, the outer shell 240 and the main support 230 continue to move downwards, which can make the first sleeve buckle 2022 of the inner sleeve 220 press against the surface of the overlapping part 2021 of the main support 230, thereby achieving the fixation of the inner sleeve 220 and the main support 230.

Furthermore, as the module support 120 has been separated from the bottom shell 111, when the reusable auxiliary implantation device 200 is picked up, the bottom shell 111 will naturally fall off, while the module support 120 and the sensor device 130 remain on the inner sleeve 220.

Next, as shown in FIG. 25, the reusable auxiliary implantation device 200 fixed with the module support 120 and the sensor device 130 is placed on the surface of the user's skin. The button body 261 is pressed downwards, and the button body 261 will push open the first sleeve buckle 2022 of the inner sleeve 220 against the overlapping part 2021, so as to release the fixed state of the inner sleeve 220 and the main support 230. Prior to this, due to the main spring 270 being in a compressed state, the main spring 270 can push the inner sleeve 220 to drive the module support 120 downwards, so that the guide needle 140 with the sensor device 130 can pierce the user's skin.

When the main spring 270 pushes the inner sleeve 220 down to the support hook 232 of the main support 230, the second sleeve buckle 2011 of the inner sleeve 220 pushes open the needle return buckle 2012 on the needle return support 210, thereby releasing the fixed state between the needle return support 210 and the inner sleeve 220.

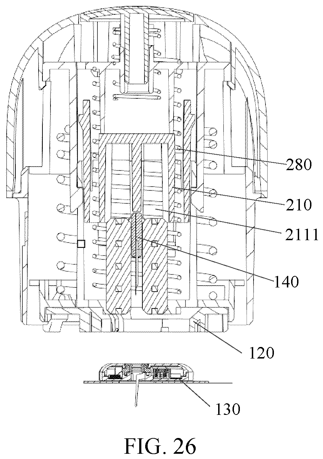

As shown in FIG. 26, when the needle return support 210 is fixed relative to the inner sleeve 220, the needle return spring 280 is in a compressed state. At this point, the needle return spring 280 can push the needle return support 210 upwards, while pulling out the guide needle 140 from the user's skin and leaving the sensor 133 inside the user's body.

And due to the dual action of the user's body and the main spring 270, the inner sleeve 220 is relatively fixed under the support of the second sleeve buckle 2011 and the support hook 232. At this point, the main spring 270 exerts a downward pressing force on the inner sleeve 220, causing the application adhesive tape 136 at the bottom of the sensor device 130 to adhere to the surface of the human body. When the reusable auxiliary implantation device 200 is removed upwards, the positive buckle 121 disengages from the positive slot 1301, thereby separating the module support 120 from the sensor device 130, leaving the sensor device 130 in the human body and the module support 120 on the inner sleeve 220.

Next, the lower cover 250 of the reusable auxiliary implantation device 200 is installed on the outer shell 240 again. The lower cover buckle 252 on the lower cover 250 is engaged with the snap-fit structure 127 of the module support 120. Then, the lower cover 250 is removed, together with the module support 120.

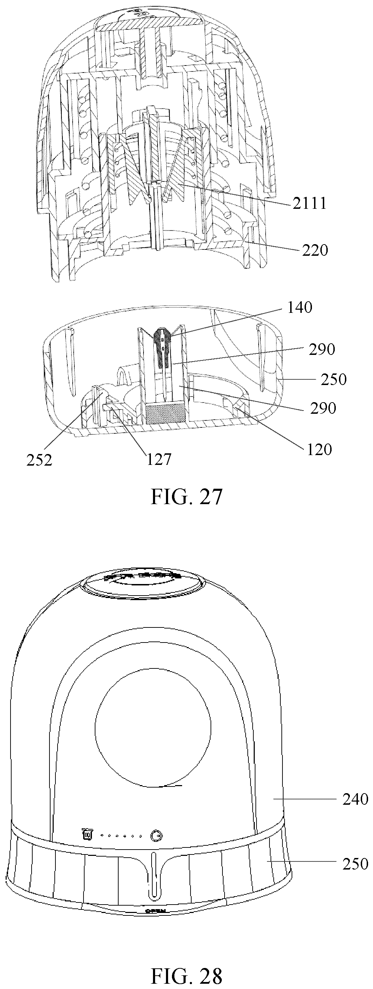

As shown in FIG. 27, when the bottom shell 111 is provided with a needle return mandril 290, and the lower cover 250 is closed to the outer shell 240, the needle return mandril 290 will expand apart the two clamping rods 2111, allowing the guide needle 140 to naturally fall into the bottom shell 111.

As shown in FIG. 21, when the button mandril 264 is provided on the button module 260, the outer shell 240 can be rotated relative to the main support 230, so that the outer shell 240 and the button module 260 are in the second gear. Then, the button body 261 is pressed again, causing the button body 261 to move downwards a longer distance. During this process, the button mandril 264 will spread apart the two clamping rods 2111, causing the guide needle 140 to naturally fall off.

Finally, as shown in FIG. 28, the lower cover 250 is once again closed to the outer shell 240. At this point, the reusable auxiliary implantation device 200 is restored to its original state before use. Operation can be conducted according to the above steps for next use.

Thus, in the embodiment of the present application, the reusable auxiliary implantation device 200 and the sensor module 100 are designed as two independent structures. When in use, the module support 120 is assembled with the inner sleeve 220. After use, the module support 120 is removed from the inner sleeve 220 using the lower cover 250 or the button module 260 to achieve the reuse of the reusable auxiliary implantation device 200, thereby reducing product costs and protecting the environment.

It will be apparent to those skilled in the art that various amendments and variations may be made to the embodiment of the present application without departing from the spirit and scope of the present application. In this way, if these amendments and variations of the present application are within the ranges of the claims of the present application and equivalent technologies thereof, the present application has also intended to contain those amendments and modifications.

## Claims

1. A sensor implantation mechanism, **characterized by** comprising a sensor module and a reusable auxiliary implantation device, wherein:
the sensor module comprises a packaging box, a module support, a guide needle, and a sensor device, the packaging box comprises a bottom shell and a box cover, and the box cover is detachably closed to the top of the bottom shell;
wherein the module support, the guide needle, and the sensor device are arranged inside the bottom shell, and the module support is detachably connected to the bottom shell; the sensor device is detachably connected to the module support, and the guide needle is detachably installed on the sensor device;
wherein the reusable auxiliary implantation device comprises an injection structure and a reset structure, the injection structure comprises a first engagement structure, and the module support comprises a second engagement structure; the first engagement structure is configured to engage with the second engagement structure to fix the module support to the injection device, and the first engagement structure is configured to drive the module support to detach from the bottom shell;
the injection structure is configured to move along an injection direction for driving the sensor device to be implanted into a human body; the injection structure also is further configured to move in an opposite direction of the injection direction for driving the module support to detach from the sensor device such that the sensor device stays in the human body;
wherein the injection structure comprises a clamping component, which is configured to clamp the guide needle; when the injection structure moves in the opposite direction of the injection direction, the injection structure is further configured to drive the guide needle to detach from the sensor device;
wherein the reset structure is configured to drive the second engagement structure to detach from the first engagement structure such that the module support detaches from the injection structure, and the reset structure is further configured to drive the guide needle to detach from the clamping component.

2. The sensor implantation mechanism according to claim 1, **characterized in that**, the reusable auxiliary implantation device further comprises an outer shell, a lower cover, and a main support; the outer shell is sleeved and connected to the main support, and the lower cover detachably closes the bottom of the outer shell;
wherein the injection structure comprises a needle return support, an inner sleeve sleeved and connected to the needle return support, and a button module;
wherein the engagement structure is arranged on an inner wall of the inner sleeve;
wherein the main support is sleeved on the inner sleeve, and a first limiting structure is provided between the inner sleeve and the main support; when the first limiting structure is in a locked state, the inner sleeve is relatively fixed to the main support; when the first limiting structure is in an unlocked state, the inner sleeve is configured to move relatively to the main support along an axis direction of the inner sleeve, wherein the axis direction of the inner sleeve is consistent with the injection direction;
wherein the button module is arranged on the outer shell, and the button module comprises a button body; the button body is configured to move relatively to the outer shell along the axis direction of the inner sleeve for driving the first limiting structure to switch from the locked state to the unlocked state such that the inner sleeve is configured to move relatively to the main support and the sensor device is configured to be driven and implanted into the human body.

3. The sensor implantation mechanism according to claim 1, **characterized in that**, the first engagement structure comprises a top rib arranged on the inner wall of the inner sleeve, and the top rib is arranged near the bottom of the inner sleeve;
wherein the second engagement structure comprises a first support buckle arranged on the module support, and when the first engagement structure is engaged with the second engagement structure, the first support buckle is pressed against the top rib.

4. The sensor implantation mechanism according to claim 3, **characterized in that**, the reset structure comprises a lower cover buckle located inside the lower cover, and the module support is provided with a snap-fit structure;
wherein when the first engagement structure is engaged with the second engagement structure, the lower cover is configured to be assembled with the outer shell from the bottom of the outer shell such that the lower cover buckle is held against the snap-fit structure;
wherein when the lower cover is detached from the outer shell in a state where the lower cover buckle is held against the snap-fit structure, the lower cover buckle is configured to drive the first support buckle to detach from the top rib such that the module support detaches from the inner sleeve.

5. The sensor implantation mechanism according to claim 3, **characterized in that**, the inner wall of the bottom shell is provided with a clamping structure, which is configured to engage with the second engagement structure to fix the module support relative to the bottom shell;
wherein when the first engagement structure is engaged with the second engagement structure, the first engagement structure is configured to drive the clamping structure to detach from the clamping structure such that the module support detaches from the bottom shell.

6. The sensor implantation mechanism according to claim 2, **characterized in that**, the clamping component comprises two clamping rods, which are symmetrically arranged according to the axis of the needle return support, and a first end of the clamping rod is connected to the inner wall of the needle return support;
wherein the clamping rod is inclined to the axis of the needle return support such that second ends of the two clamping rods are close to each other and the second ends of the two clamping rods are configured to corporately clamp the guide needle.

7. The sensor implantation mechanism according to claim 6, **characterized in that**, the reset structure comprises a needle return mandril located inside the lower cover; when the first engagement structure is engaged with the second engagement structure, the lower cover is configured to be assembled with the outer shell from the bottom of the outer shell; the needle return mandril is configured to drive the second ends of the two clamping rods to move away from each other such that the guide needle detaches from the clamping component.

8. The sensor implantation mechanism according to claim 6, **characterized in that**, the reset structure comprises a button mandril connected to the button body, and the button mandril extends along the axis direction of the inner sleeve;
wherein when the button body moves along the axis direction of the inner sleeve, the button mandril is configured to extend between the two clamping rods and drive the second ends of the two clamping rods to move away from each other such that the guide needle detaches from the clamping component.

9. The sensor implantation mechanism according to claim 8, **characterized in that**, a first gear and a second gear are provided between the button module and the outer shell;
wherein when the button module and the outer shell are in the first gear, the button module is configured to drive the first limiting structure to switch from the locked state to the unlocked state;
wherein when the button module and the outer shell are in the second gear, the button module is configured to drive the second ends of the two clamping rods away from each other.

10. The sensor implantation mechanism according to claim 9, **characterized in that**, the button module further comprises a button limiting rib, and the inner wall of the outer shell comprises a button limiting table;
wherein the outer shell is configured to rotate around the axis of the main support relative to the button module; when the button module and the outer shell are in the first gear, the button limiting table is located below the button limiting rib; when the button module and the outer shell are in the second gear, the button limiting table is offset from the button limiting rib along a circumference of the outer shell.

11. The sensor implantation mechanism according to claim 2, **characterized in that**, a main spring is connected between the main support and the inner sleeve, and when the first limiting structure is in a locked state, the main spring is in a compressed state.

12. The sensor implantation mechanism according to claim 2, **characterized in that**, a second limiting structure is provided between the needle return support and the inner sleeve; when the second limiting structure is in a locked state, the needle return support and the inner sleeve are fixed relatively to each other; when the second limiting structure is in an unlocked state, the needle return support is configured to move relatively to the inner sleeve along the axis direction of the inner sleeve.

13. The sensor implantation mechanism according to claim 11, **characterized in that**, the inner wall of the main support comprises a third limiting structure, which is located near the bottom of the main support;
wherein the third limiting structure is configured to limit the movement distance of the inner sleeve relative to the main support, and is further configured to drive the second limiting structure to switch from the locked state to the unlocked state.

14. The sensor implantation mechanism according to claim 12, **characterized in that**, the second limiting structure comprises a needle return buckle arranged on the outer wall of the needle return support and a second sleeve buckle arranged on the inner sleeve, and when the second limiting structure is in a locked state, the needle return buckle is pressed against the bottom of the second sleeve buckle;
wherein the third limiting structure comprises a support hook arranged on the inner wall of the main support; when the inner sleeve moves away from the button module relative to the main support, the support hook is configured to drive the needle return buckle for releasing from the second sleeve buckle and pressing the support hook against the bottom of the first sleeve buckle.

15. The sensor implantation mechanism according to claim 13, **characterized in that**, a needle return spring is connected between the inner sleeve and the needle return support; when the second limiting structure is in a locked state, the needle return spring is in a compressed state.

16. The sensor implantation mechanism according to claim 2, **characterized in that**, the inner sleeve comprises a sleeve limiting rib, and the module support comprises a limiting groove; when the first engagement structure is engaged with the second engagement structure, the sleeve limiting rib is located in the limiting groove.

17. The sensor implantation mechanism according to claim 1, **characterized in that**, the module support comprises a positive buckle and a negative buckle, and the sensor device comprises a positive slot and a negative slot;
wherein the positive buckle is configured to be clamped to the positive slot, and the negative buckle is configured to be clamped to the negative slot; the negative buckle and the negative slot are configured to restrict the sensor device from coming out of the module support in the opposite direction of the implantation direction of the sensor device.

18. The sensor implantation mechanism according to claim **1, characterized in that**, the guide needle comprises a plastic handle and a puncture needle;
wherein the sensor device comprises a sensor bottom shell, a sensor support, and a sensor; the sensor support is arranged on the sensor bottom shell, and the sensor is clamped between the sensor support and the sensor bottom shell;
wherein the guide needle passes through the sensor support and the sensor bottom shell such that the puncture needle is located on the side of the sensor bottom shell facing away from the sensor support; the surface of the plastic handle facing the puncture needle is flush with the surface of the sensor bottom shell facing away from the sensor support.

19. The sensor implantation mechanism according to claim 17, **characterized in that**, the sensor support comprises a perforation, the plastic handle passes through the perforation, and a sealing rubber ring is provided between the plastic handle and the perforated inner wall of the sensor support.

20. The sensor implantation mechanism according to claim 17, **characterized in that**, a sealing column is provided in the bottom shell, and the sealing column butts against the surface of the sensor bottom shell facing away from the sensor support;
wherein an opening is provided at one end of the sealing column butting against the sensor bottom shell, and the puncture needle is configured to be extended into the sealing column through the opening.
